# EUROPEAN PATENT APPLICATION

(11) **EP 4 218 750 A1**
(43) Date of publication of application: **02.08.2023**
(21) Application number: 22154467.9
(22) Date of filing: 01.02.2022
(51) Int. Cl.: A61K 31/198, A61K 31/404, A61K 31/787, A61P 25/28

(54) **THE USE OF MELANIN IN THERAPY OF SUBJECTS WITH TRISOMY 21**

(71) Applicant: Solis Herrera, Arturo, CP20000 Aguascalientes (MX)
(72) Inventor: Solis Herrera, Arturo, CP20000 Aguascalientes (MX)
(74) Representative: Bals & Vogel Patentanwälte PartGmbB

(57) **Abstract**

The present invention relates to a substance, and a tablet for use in therapy of subjects with Trisomy 21. Furthermore, the invention relates to a dispensing device for use with said substance or tablet.

## Description

The present invention relates to a substance, and a tablet for use in therapy of subjects with Trisomy 21. Furthermore, the invention relates to a dispensing device for use with said substance or tablet.

Trisomy 21 (also known as Down syndrome) in human subjects is a genetic disorder caused by the complete or partial triplication of chromosome 21, which can lead to physical growth delays, intellectual disability (in particular reduced IQ and/or mental impairment), characteristic facial features and/or congenital heart diseases (found in approximately 40 to 50% of subjects). Further clinical manifestations and/or risks can comprise a short neck, an increased amount of skin on the back of the neck, a low muscle tone, flexible ligaments, a narrow roof of the mouth, enlarged hypotonic tongue, crusted and hypotonic lips, a flat head, a protruding tone, abnormal outer ears, a flattened nose, abnormal teeth, in particular Gingivitis, slanted eyes, occurrence of Brushfield spots in the iris, strabismus, shortened hands, obstructive sleep apnea, single transverse palmar crease, congenital heart disease, hypothermia, leukemia (which is 10 to 15 times more common in infants with down syndrome), hypothyroidism, constipation, infertility, undescended testicles, Alzheimer's disease and/or accelerated aging. The life expectancy of human subjects with Trisomy 21 is around 50 to 60 years in the developed world when proper health care is provided. The occurrence of Trisomy 21 is about 1 in 1000 babies born each year. About 92% of pregnancies in Europe with a diagnosis of Trisomy 21 are terminated. If at least one of the parents has Trisomy 21, there is an increased chance that the baby will also have Trisomy 21. However, Trisomy 21 can also occur for parents which are genetically normal, with an increasing probability depending on the age of the mother. The extra chromosome is believed to occur by chance, with no known behavioral activity or environmental factor that changes the probability.

In the prior art, there seems to be little to no effort with respect to treatment of Trisomy 21, in particular for newborn babies and infant human subjects (up to around 5 years of age). Also, prenatal treatment, when the baby is still unborn, in particular after diagnosing Trisomy 21, is still problematic. Treatment of Trisomy 21 so far has been ineffective and unspecific. The development and/or influence of Trisomy 21, in particular regarding the aforementioned parameters can consequently be improved.

Thus, it may be one object of the present invention to optimize the treatment of Trisomy 21, in particular by reducing and/or mitigating the aforementioned disadvantages for subjects.

In general, and in the context of the present invention in particular, a substance and/or tablet can be administered to a subject. In other words, it is given to the subject, in particular by administration, wherein several ways of administration are described below, which can be used exclusively or in any combination. After administration, the substance can essentially enter the blood of the subject, in particular it can enter the bloodplasma and be distributed at least partly inside the subject or the subject's body. In other words, the substance can be administered and/or distributed comparably to a drug or medicine. The substance can be metabolized and/or excreted, in particular subsequent to administration. These mechanisms can be part of and/or described by the pharmacokinetics of the substance. Pharmacokinetics can comprise liberation, absorption, distribution, metabolism and/or excretion, preferably all of the steps (also known as "LADME"). Liberation can comprise a process of release of the substance from the tablet or pharmaceutical formulation, which can comprise all substances or materials comprised therein. Absorption can comprise a process of a substance entering the blood, in particular entering the blood circulation and/or blood plasma. Distribution can comprise a dispersion or dissemination of a substance throughout the fluids and tissues of the subject, in particular via distribution of blood and/or blood plasma in the subject's body. Metabolism can comprise a process of recognition by the subject's organism that a substance is present and, in particular, metabolism can comprise the irreversible transformation of parent compounds comprised by the substance into daughter metabolites. Excretion can comprise a removal of the substance from the subject. In other words, the substance is at least partly excreted by the subject, for example via the digestive tract. Plasma concentration can describe the amount of the substance in the blood plasma, in particular related to the weight or volume in a given plasma volume. Plasma half-life (also known as elimination half-life) describes the time that elapses after administration, in particular intravenous administration, between the maximum plasma concentration of a substance in the blood plasma and the fall to half of this value. The plasma half-life can depend on numerous factors influencing the circulating blood in the subject, in particular the substance can be metabolized in the liver, accumulated in tissues, be excreted by the kidney, and/or cross the blood-brain barrier. If the substance is metabolized by the liver, there can be an exponential progression of plasma concentration over time. Dose can describe the amount of substance administered, in particular comprising a unit of mol, g, and/or oz. Dosing interval can describe the time between two administrations. In other words, the substance, in particular a dose of the substance, can be administered, then during a dosing interval of e.g. 1 hour no administration is performed, then the substance, in particular a dose of the substance, can be administered again. Time to reach maximum plasma concentration can describe the point in time between administration and a later point in time comprising the maximum plasma concentration. Absorption half-life can describe the time required for 50% of a given dose of a substance to be absorbed into the blood, in particular the blood plasma and/or systemic circulation. Effect can describe the impact of the substance on the subject. In general, a relationship between the plasma concentration and the effect can be observed, in particular a substance and/or tablet can influence the plasma concentration (e.g. displayed along an x-axis of a coordinate system) and effect (e.g. displayed along a y-axis of said coordinate system) at least in parts. For example, the plasma concentration and effect, in particular the relationship between them, can be at least partly linear. In other words, a raise of 50% in plasma concentration can lead to a 50% raise in effect. For example, the relationship can approximately comprise at least partly a hysteresis and/or exponential relationship. If at least two or more dosing intervals, in particular constant dosing intervals (e.g. equidistant in time) are used to administer the substance, the relationship between plasma concentration and effect can comprise an essentially cyclical process, wherein preferably following (a first) administration the effect increases when the plasma concentration increases, the effect decreases when the plasma concentration decreases following the maximum plasma concentration, and the process is repeated following a subsequent (second) administration after a dosing interval has essentially elapsed. This process can be maintained by repetition, which can particularly lead to a steady state between plasma concentration (e.g. displayed on a y-axis) and time (e.g. displayed on an x-axis). Minimum steady state concentration can comprise the lowest plasma concentration, in particular which is not fallen short of during steady state. Average steady state concentration can comprise the average plasma concentration, in particular which comprises the mean (or averaged) value of plasma concentration over time during steady state. The time during which the substance is active inside the body of the subject can be described as duration of action. In other words, this can describe the time until the substance is completely removed from the subject, e.g. by excretion. The substance can have a local effect or a global effect on the whole subject. The substance can also have an increased effect locally and a reduced effect more distant from a local spot, in particular a local spot, in particular a location where the administration was performed e.g. by injection, oral administration and/or adhesive administration. This can be beneficial to provide an increased effect at a specific location, in particular a body part of the subject.

According to a first aspect of the invention, a substance, analogues thereof, precursors thereof, or its derivates, for use in the treatment of Trisomy 21 are provided. In particular, the substance may comprise analogues thereof, precursors thereof, or its derivates, in other words, the substance can be a mixture thereof. The substance can be for use as a medicament. The substance may be for use in a method for the treatment of Trisomy 21. In particular, the substance, analogues thereof, precursors thereof, or its derivates can be for use in the therapy of Trisomy 21, in particular in therapy of subjects with Trisomy 21. This can comprise administration of the substance, analogues thereof, precursors thereof, or its derivates to the subject with Trisomy 21.

The substance comprises C₁₇H₂₀N₂O₃ (melanin), a compound A (in particular 3-(1-Methylpyrrolidin-2-yl)pyridine, 3-[(2S)-1-Methyl-2-pyrrolidinyl]pyridine, (S)-3-[1-Methylpyrrolidin-2-yl]pyridine and/or C₁₀H₁₄N₂) or a pharmaceutically acceptable substitute in particular a salt thereof 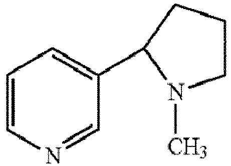 and/or a compound B (in particular salicylic acid, phenol-2-carboxylate, HOC₆H₄COOH and/or C₇H₆O₃) or a pharmaceutically acceptable substitute in particular a salt thereof

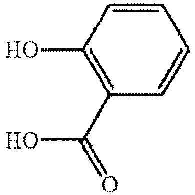

The substance can be used for early, more robust, more effective, more reliable, easier to produce and/or cheaper use in the treatment of Trisomy 21. The substance can have an impact, in particular a positive medical impact, on a tricarboxylic acid cycle (TCA) in the subject, in particular essentially inside the body of the subject This can influence, in particular at least partly reduce, the evolution and/or severity of Trisomy 21, and/or in particular the substance can reduce the symptoms, advantageously in a more efficient way. In general, the substance can be administered (e.g. given) to a subject with Trisomy 21, in particular in some form described below. Preferably, the substance can pass the blood-brain barrier of a subject, in particular to provide an improved effect. The substance can comprise or can be an active substance. The substance can be used alone or in combination with other active substances, wherein a substance and/or active substance can be a biologically active substance which comprises a beneficial or adverse effect on a subject, in particular living matter, in particular on human subjects. In the present invention, a subject can be a human or a human fetus, in particular a woman, pregnant woman, fetus, baby, infant, child, teenager, or man. In particular the substance can be used independent of the gender. The substance can comprise a plasma half-life between 0.1 and 72 hours, advantageously between 0.5 and 24 hours, preferably between 1 and 6 hours, more preferably between 2 and 5 hours, ideally between 3 and 4 hours. A plasma half-life between 3 and 4 hours has been found to be particularly advantageous to achieve a beneficial average steady state concentration, wherein the average steady state concentration can comprise the average plasma concentration at steady state describing the total exposure over 1 dosing interval divided by the time of the dosing interval. While concentrations can rise and fall during a dosing interval at steady state, the average concentration does not change. The substance can comprise a plasma half-life between 10 and 300 minutes, advantageously between 30 and 120 minutes, preferably between 55 and 95 minutes, more preferably between 65 and 85 minutes, ideally between 70 and 80 minutes, in particular for the use in treatment of Trisomy 21 in a fetus and/or infant. Longer plasma half-life, in particular between 80 and 300 minutes, can provide an increased effect, preferably when longer dosing intervals are used. Shorter plasma half-life, in particular between 10 and 70 minutes, can provide an increased effect, preferably when shorter dosing intervals are used and/or a faster effect on the subject is desired.

Another aspect of the invention can be to provide a substance for use in the treatment of Trisomy 21, wherein the substance is designed for postnatal administration, in particular of newborn babies and infants, and/or for prenatal treatment of a pregnant human and/or the fetus, in particular the unborn baby. In other words, the substance can be given to children and/or the mother before birth of her baby. This can be advantageous, since the effectiveness of the treatment and/or the efficiency can improve when the administration, treatment and/or therapy is started as soon as possible, in particular after Trisomy 21 has been diagnosed. This can in particular minimize and/or mitigate the evolution and/or the symptoms of Trisomy 21. The substance can be given to the unborn baby and/or to the pregnant mother, wherein a combination can improve the success rate and efficiency, in particular for the baby, in particular due to the shared blood cycle, which preferably allows to administer the substance passively to the fetus via the mother.

Another aspect of the invention can be to provide a substance for use in the treatment of Trisomy 21, wherein the substance is essentially administered in solid, liquid or gaseous form or in an essentially hybrid form thereof. The substance can be soluble in water, preferably in blood. A solid, liquid or gaseous form can describe the state of aggregation of the substance. A solid form can be advantageous for administration, in particular for oral, nasal and/or adhesive administration, and in particular for use as or in a tablet (described below). Furthermore, a solid state of aggregation can allow very precise dosage, in particular this can prevent fluctuations of dosage among different tablets, which can result in a more robust treatment. This can also facilitate storing and/or transportation of the substance and/or tablets, in particular for large amounts thereof. The liquid form can be predominant at room temperature, while preferably heating the substance, in particular to the human body temperature, can result in the substance to become at least partially liquid, for facilitated administration e.g. by intravenous or oral administration. A liquid form can be advantageous for administration, in particular for oral administration, and/or intravenous administration, which is particularly advantageous for unconscious subjects, infants, newborn babies and/or fetuses. A gaseous form can be advantageous for administration, in particular for inhalation. A hybrid form between solid and liquid form can be advantageous for easier and/or faster oral administration, in particular it can be easily produced for example by crushing a tablet comprising the substance and then dissolving it in an aqueous solution and/or electrolytes for intravenous administration. A hybrid form between liquid and gaseous form can be advantageous for easier and/or faster nasal administration and/or inhalation, in particular if the substance is dissolved in a liquid and then mixed with a gas, for example air, before inhalation. This can allow storage of the substance in liquid form and subsequent inhalation. In the context of the present invention, a drop, particularly comprising the substance in a liquid state of aggregation, can comprise the substance in 100% pure form. A drop can comprise a volume between 0.00001 and 100 ml, advantageously between 0.0001 and 10 ml, preferably between 0.001 and 5 ml, more preferably between 0.005 and 5 ml, ideally between 0.01 and 0.1 ml. A drop can be administered per oz. (in particular fluid ounce), wherein 1 oz. can essentially correspond to 29,5735 ml. Consequently, 3 drops per oz. can correspond to 3 drops per 1 oz. of water, volume of the subject, volume of the blood volume of the subject, or volume of the blood plasma of the subject.

Another aspect of the invention can be to provide a substance for use in the treatment of Trisomy 21, wherein the compounds A and B are substantially at a ratio of between 10:1 to 1:10, by weight, advantageously at a ratio of about 1.5:1. In particular, the substance can comprise compound A and compound B mixed at a ratio of about 1:10, 1:8, 1:6, 1:4, 1:2, 1:1, 1.5:1, 2:1, 4:1, 6:1, 8:1 or 10:1, by weight. A ratio of 1.5:1 can be advantageous for oral administration, in particular due to increased efficiency, in particular for accelerated liberation, increased robust treatment and/or time to reach maximum plasma concentration. A ratio comprising an increased part of compound A, in particular compound A and compound B mixed at a ratio of about 1.5:1, 2:1, 4:1, 6:1, 8:1 or 10:1, can be advantageous due to increased metabolism, exretion and/or higher costs of compound B (e.g. to reduce the costs). A ratio comprising an increased part of compound B, in particular compound A and compound B mixed at a ratio of about 1:10, 1:8, 1:6, 1:4, 1:2, can be advantageous due to easier production, increased plasma half-life, improved distribution and/or accelerated liberation.

Another aspect of the invention can be to provide a substance for use in the treatment of Trisomy 21, wherein the substance is adapted for oral administration, for inhalation, nasal administration, intravenous administration, and/or adhesive administration, in particular as described below. In other words any one way of administration may be provided or at least two of them. This can also comprise administration according to all these aspects. The administration can result in a plasma half-life between 0.1 and 72 hours, advantageously between 0.5 and 24 hours, preferably between 1 and 6 hours, more preferably between 2 and 5 hours, ideally between 3 and 4 hours. A plasma half-life between 3 and 4 hours has been found to be particularly advantageous to achieve a beneficial average steady state concentration, wherein the average steady state concentration comprises the average concentration at steady state describing the total exposure over 1 dosing interval divided by the time of the dosing interval. While concentrations can rise and fall during a dosing interval at steady state, the average concentration does not change. The administration comprises a time to reach maximum plasma concentration between 0.1 and 3600 seconds, advantageously between 1 and 1200 seconds, preferably between 10 and 240 seconds, more preferably between 30 and 120 seconds, ideally between 45 and 75 seconds. A time to reach maximum plasma concentration between 45 and 75 seconds has been found to be particularly advantageous to achieve an effect of the substance in optimized time.

Another aspect of the invention can be to provide a substance for use in the treatment of Trisomy 21, wherein the substance is adapted for oral administration, in particular that the substance is essentially shaped as a tablet, as a suppository and/or as an essentially shaped granulate, wherein advantageously the oral administration is essentially buccal, sublabial or sublingual. In other words, the tablet, suppository and/or essentially shaped granulate can comprise at least the substance, and preferably at least one excipient and/or coating. In general, oral administration comprises swallowing the substance and/or at least partly keeping the substance in the mouth, to allow for melting and/or an absorption. The administration of the substance in general, and oral administration in particular, can benefit from a retarded release (described below). Preferably, the substance can comprise a liquid state of aggregation for oral administration. This can comprise dissolving the substance in a liquid, in particular water.

A tablet and/or suppository can be easily manufactured and/or stored. A suppository can furthermore be given to infants, newborn and/or unconscious subjects, in particular if these subjects are not able to accomplish an oral administration by themselves or with help.

A suppository can be inserted into the body, particularly in the ear, nose, mouth, rectum, vagina or urethra, where it dissolves and/or melts, in particular it is subsequently absorbed into the blood and/or blood plasma. A suppository can be used to deliver the substance both systemically (e.g. global to the whole body) and locally (e.g. for a local spot). Preferably, a suppository comprises a greasy base, like cocoa butter, and/or a water soluble base, such as polyethylene glycol, and/or glycerol and gelatin, which can melt at body temperature. A suppository can be easier to administer compared to a tablet, particularly when a subject has a vomiting tendency and/or is unable to administer the substance and/or tablet otherwise (e.g. by itself). The substance can be better tolerated in suppository form. A suppository can be advantageous since no digestion is required, and particularly the substance can be administered in the vicinity of the target tissue. A suppository can be preferably compared to a tablet or oral administration because a reliable intake occurs.

A granulate can preferably be mixed with food, such that preferably the substance is gradually absorbed over a longer period of time, in particular via the stomach and or the digestive tract, for example within at least 30 minutes. Oral administration, and a granulate in particular, can be advantageous to achieve a certain time profile of absorption in the subject, in particular as described above, and can particularly be well-suited for adults and children older than 5 years. Furthermore, oral administration, and a granulate in particular, can be achieved without further assistance and/or technical devices, in particular for continuous treatment outside the clinical environment or medical care. A granulate can also make the oral administration easier and/or more digestible.

Another aspect of the invention can be to provide a substance for use in the treatment of Trisomy 21, wherein the substance is intended for inhalation. Inhalation can comprise that the subject essentially inhales the substance, in particular in liquid and/or gaseous form, preferably to transport the substance to the lung of a subject. This can be advantageous since the absorption is at least partly or totally passive. In other words, the substance can be provided essentially without the active help of the subject, which can be advantageous for babies, infants, disabled and/or unconscious subjects. This can furthermore be beneficial for subjects which can hardly or cannot accomplish oral administration, in particular due to pain related to the mouth or jaw. Furthermore, the absorption can be accomplished via mucous membranes and/or the lungs, which is particularly fast compared to oral administration. For inhalation, an inhaling device can be used. This allows for additional drugs and/or substance to be taken essentially at the same time (e.g. in a mix) or taken before or afterwards. Inhalation also allows for a precise dosage and/or a changing dosage. The substance can also be mixed with other gases, for example air, oxygen et cetera. Air and oxygen can be advantageous for short-breathed and/or subjects in an emergency.

Another aspect of the invention can be to provide a substance for use in the treatment of Trisomy 21, wherein the substance is intended for nasal administration. This can comprise that the subject essentially inhales the substance, in particular in liquid and/or gaseous form, for example using a using an inhaler device, in particular a portable inhaler device. Nasal administration can be advantageous since the absorption is at least partly or totally passive. In other words, the substance can be provided essentially without the active help of the subject, which can be advantageous for babies, infants, disabled and/or unconscious subjects. This can furthermore be beneficial for subjects which can hardly or cannot accomplish oral administration, in particular due to pain related to the mouth or jaw. Nasal administration can also comprise that the subject uses the substance at least partially in liquid and/or solid form. This can comprise using a substantially gel-like substrate, advantageously such as an ointment, lotion, cream, gel, or tincture, in particular as described below for an adhesive administration. Furthermore, the absorption can be accomplished via mucous membranes and/or the lungs, which is particularly fast compared to oral administration.

Another aspect of the invention can be to provide a substance for use in the treatment of Trisomy 21, wherein the substance is adapted for intravenous administration. This can preferably comprise the intravenous administration into the vascular system of a subject, advantageously comprising the substance in liquid and/or solid form, preferably using conventional instruments for intravenous administration, in particular comprising a needle for piercing the skin and entering a vein which is connected to a syringe or to external tubing comprising at least the substance. Intravenous administration can be advantageous since the absorption is at least partly or totally passive. In other words, the substance can be provided essentially without the active help of the subject, which can be advantageous for babies, infants, disabled and/or unconscious subjects, in particular subjects with an inactive cardiopulmonary system. This can furthermore be beneficial for subjects which can hardly or cannot accomplish oral administration, in particular due to pain related to the mouth or jaw. Intravenous administration can be the most robust, reliable and/or fastest way to provide a substance to a subject, since the substance is directly injected in the vascular system, dissolved and/or mixed with blood, in particular blood plasma, and distributed to all body parts. In other words, the time to reach maximum plasma concentration is minimized. The dose can precisely be set and/or changed depending on the subject. Furthermore, it can be assured, that the substance reaches the vascular system, in particular since it is directly injected therein, which can be advantageous for supervisors such as clinical staff, who have to ensure that a certain substance is safely and/or reliably administered. Intravenous administration can comprise administration of additional medications and/or other medical therapy such as blood products or electrolytes to correct electrolyte imbalances. Intravenous administration can comprise administration as a dose and/or bolus rapidly (almost instantly) or may be administered slowly, in particular at a constant rate, between 5 and 600 seconds, preferably between 10 and 300 seconds, especially preferred between 20 and 180 seconds, ideally between 30 and 120 seconds. In the context of the present invention it has been found out that an intravenous administration administered in between 45 and 60 seconds is particularly effective, in particular for the therapy of infants. Intravenous administration can be particularly advantageous for prenatal treatment of a pregnant human and/or the unborn baby, especially as long as the baby is still connected to the vascular system of the mother. In addition or alternatively, intravenous administration can comprise infusion to achieve an approximately constant plasma concentration of the substance, in particular the infusion can be administered during a time interval between 0.5 and 72 hours, preferably between 2 and 48 hours, especially preferred between 4 and 36 hours, ideally between 12 and 24 hours. Finally, intravenous administration allows to easily mix the substance with other drugs, medication, nutrition, electrolytes and/or active substances. The substance can be administered by intravenous administration into the umbilical cord, the amniotic fluid, the body of the newborn baby, infant, pregnant human and/or the fetus, in particular the unborn baby. The substance can be administered via a venous access of the subject, in particular the arm of the subject, preferably for adult subjects. An infusion rate, in particular to balance elimination of the substance can comprise between 0.01 to 1000 mmol/h, advantageously between 1 to 250 mmol/h, preferably between 10 to 130 mmol/h, more preferably between 20 to 100 mmol/h, ideally between 30 to 75 mmol/h. Higher values, in particular between 75 to 1000mmol/h, can provide an improved effect of the substance for adult subjects. Lower values, in particular between 0.01 to 30 mmol/h, can provide an improved effect of the substance for infant subjects or a fetus.

Another aspect of the invention can be to provide a substance for use in the treatment of Trisomy 21, wherein the substance is adapted for adhesive administration, in particular for adhesive administration comprising a substantially gel-like substrate, advantageously such as an ointment, lotion, cream, gel, or tincture, which can directly be applied and/or rubbed on or in the skin. Adhesive administration can be advantageous since the absorption is at least partly or totally passive. In other words, the substance can be provided essentially without the active help of the subject, which can be advantageous for babies, infants, disabled and/or unconscious subjects. This can furthermore be beneficial for subjects which can hardly or cannot accomplish oral administration, in particular due to pain related to the mouth or jaw. For adhesive administration, the absorption can be delayed, since the substance has to pass the skin which acts as a barrier. This also allows to administer the substance locally, in particular only on certain body parts, preferably the face, mouth, hands or feet. It is also possible to combine and/or mix the substance with at least one other gel-like substrate, preferably with a sun cream and/or body lotion. This can particularly improve the melanin level in the subject and/or can provide increased melanin production.

Another aspect of the invention can be to provide a substance for use in the treatment of Trisomy 21, wherein the substance affects nAch receptors, in particular such that the substance at least partly couples to said nAch receptors, and/or stimulates the said nAch receptors. Nicotinic acetylcholine receptors (nAChR) comprise neuronal receptor proteins that signal upon a chemical stimulus which can be provided by the substance. The nAChR can form ligand gated ion channels in the plasma membrane of a certain neuron. They can be expressed on the presynaptic and postsynaptic side of the neuro muscular junctions. They can be directly linked to ion channels and thereby are not dependent on second messengers. The nAChR can be found in the central nervous system as well as in the peripheral nervous system of subjects, in particular mammalians like humans. Generally, ligand gated ion channels require the binding of a chemical messenger for opening, wherein an endogenous agonist for opening can be acetylcholine, wherein they are also triggered by the substance, analogues thereof, precursors thereof or its derivatives, in particular (S)-3-[1-Methylpyrrolidin-2-yl]pyridine. The action that the substance, and (S)-3-[1-Methylpyrrolidin-2-yl]pyridine in particular, binds to nAChR can be regarded as a key energy source of the cell, as this can be responsible for upregulating of energy. The nAChR can produce excitatory postsynaptic effects. Thereby, energy can be upregulated previous to an excitatory postsynaptic effect. Further, the administration of the substance, analogues thereof, precursors thereof or its derivatives, in particular (S)-3-[1-Methylpyrrolidin-2-yl]pyridine, can upregulate the release of an endogenous alpha-MSH. The melanocytes stimulating hormone (MSH) belong to peptide hormones that are produced in the central nervous system. These hormones can stimulate the production inducing melanin by melanocytes in the skin and in the hair. Consequently, an adhesive administration can be advantageous in particular due to a local production in the skin. Alpha-MSH can induce multiple responses in nearly all cells of a subject, thereby significantly improving and also maintaining good health conditions. Alpha-MSH can further be related to chemical energy levels within the subject's body, and is thereby fundamental for an optimal body performance. Alpha-MSH can also reduce or prevent Trisomy 21, and/or the symptoms of Trisomy 21.

Another aspect of the invention can be to provide a substance for use in the treatment of Trisomy 21, wherein the substance is designed for decreasing or increasing the subject's core body temperature or the temperature of at least one body part of the subject, in particular for hypothermia. Subjects with Trisomy 21 can potentially have reduced body temperature, in particular body core temperature. The substance can decrease or increase the core body temperature, in particular by oral, nasal, intravenous administration and/or inhalation. The substance can also decrease or increase the temperature of at least one body part of the subject, in particular by local administration, advantageously by adhesive administration. The temperature can be measured before and after administration and/or therapy in order to adjust the correct dose of the substance, dosing interval and/or administration.

Another aspect of the invention can be to provide a substance for use in the treatment of Trisomy 21, wherein the substance is designed to change at least one of the following parameters, in particular to increase, strength, muscle tone, attention, activity, memory, and/or cognition. Subjects with Trisomy 21 can have reduced strength, muscle tone, attention, activity, memory, and/or cognition. By administration of the substance to such subjects, these parameters can be at least partly, and preferably completely, increased, in particular such that the parameters are normal and/or comparable to healthy subjects of the same age.

Another aspect of the invention can be to provide a substance for use in the treatment of Trisomy 21, wherein the substance is designed to change and/or changes at least one of the following parameters, in particular to increase or accelerate, the rate of cell growth or cell division in order to change the proportion of abnormal cells compared to normal cells, in particular that the amount of normal cells is higher than the amount of abnormal cells. Subjects with Trisomy 21 can comprise an increased amount of abnormal cells, in particular when the disease is more pronounced and/or the symptoms are more severe. Trisomy 21 and/or symptoms thereof can be healed and/or reduced by reducing the amount of abnormal cells compared to the normal cells. The treatment of young subjects and/or a fetus can be advantageous, since the cells in these subjects are multiplying at accelerated rates. Consequently, an early administration of the substance can be beneficial. Furthermore, the complete or partial triplication of chromosome 21 can be reduced, the earlier a therapy is performed. Consequently, an increased impact on the amount of normal and/or abnormal cells can be provided and better therapy results can be achieved compared to an adult subject.

Another aspect of the invention can be to provide a substance for use in the treatment of Trisomy 21, wherein the substance is designed to change at least one of the following parameters, in particular to reduce or heal, tiredness, crying, sickness, macroglossia, hypotonia, cognitive dysfunction, cognitive impairment, congenital heart diseases, mental impairment, stunted growth, increased skin on back of the neck, roof of mouth, flat head, flexible ligaments, protruding tone, abnormal outer ears, flattened nose, abnormal teeth, slanted eyes, short neck, shortened hands, obstructive sleep apnea, single transverse palmar crease, Brushfield spots in the iris, strabismus, undescended testicles, leukemia, Hypothyroidism, Constipation, Gingivitis, Infertility, Alzheimer's disease, accelerated aging, cyanotic color or bruising or crusting or hypotonic tendencies of body parts, in particular of the lips, the fingers, and the nails.

Another aspect of the invention can be to provide a substance for use in the treatment of Trisomy 21, wherein the substances impacts a tricarboxylic acid cycle (TCA) of the subject, in particular to ease and/or cure Trisomy 21 and/or the symptoms of Trisomy 21, wherein in particular the breakdown of organic fuel molecules in the presence of oxygen is essentially improved. This can increase the energy level of a cell, in particular of cells in the subject. It can be advantageous to provide an increased concentration of oxygen, in particular by inhalation, to increase the effect.

Another aspect of the invention can be to provide a substance for use in the treatment of Trisomy 21, wherein the mRNA level is changed, in particular to ease and/or cure Trisomy 21 and/or the symptoms of Trisomy 21, in particular that the mRNA level is increased or decreased.

Another aspect of the invention can be to provide a substance for use in the treatment of Trisomy 21, wherein Trisomy 21 phenotypes like APP, BACH1, TIAM1, SOD1, SYNJ1, OLIG1, OLIG2, IFNAR1, IFNAR2, IFNGR2, GART, ITSN1, DSCR1, CBR1, CBR3, DOPEY2, MORC3, CLDN14, SIM2, HLCS, PIGP, TTC3, DSCR3, DYRK1A, KCNJ6, ERG, Ets2, HeMGN1, PCP4, DSCAM, BACE2, and S100β are changed, in particular that their amount is increased or decreased.

Another aspect of the invention can be to provide a substance for use in the treatment of Trisomy 21, wherein the substance is adapted for retarded release. Therefore, the substance and/or the tablet can comprise an excipient, in particular for providing a retarded release, preferably for the purpose of slower liberation of the substance, in particular from a tablet, long-term stabilization, bulking up solid formulations that contain potent active ingredients in small amounts (thus often referred to as "bulking agents", "fillers", or "diluents"), and/or to confer a therapeutic enhancement on the substance, in particular in the final dosage form, such as facilitating absorption, reducing viscosity, and/or enhancing solubility. Due to the delayed release of the substance, the dosing interval can be extended and the substance has to be administered correspondingly less frequently, which can have a positive impact on adherence to therapy. Retarded release can particularly be attractive for a substance and/or tablet comprising a short plasma half-life. Retarded release of a substance can be used to ensure that the effect is achieved in a delayed, prolonged, continuous and/or controlled manner over a longer period of time. This allows the timing, location (e.g. in the stomach or intestine) and/or speed of release to be influenced. Excipients can also be advantageous in the manufacturing process, to aid in the handling of the substance concerns such as by facilitating powder flowability or non-stick properties, and/or in addition to aiding in vitro stability such as prevention of denaturation or aggregation over the expected shelf life. The selection of appropriate excipients can also depend upon the administration and the dosage form, as well as the substance or active ingredient and other factors. A retarded release can result in a retarded raise of the plasma concentration. Thus, a retarded release can provide that the substance is not absorbed at once and/or at the beginning directly following administration, but delivered in a delayed way, in particular at least at two points in time and/or over a longer period of time, wherein the time during which the substance is active inside the body of the subject can be described as duration of action. In other words, the retarded release can lead to a dampened and/or widened curve, in particular regarding the relation between time (e.g. in hours shown on the x-axis of a coordinate system) and blood concentration (e.g. in µg/ml shown on the y-axis of said coordinate system). Retarded release can also lead to a reduced maximum plasma concentration, which can prevent problems occurring due to high plasma concentration like e.g. a shock. Advantageously a retarded release ensures that a single intake and/or administration of the substance leads to an absorption over a duration of action, in particular to an increased duration of action, wherein the duration of action can be between 10 minutes and 96 hours, preferably between 30 minutes and 48 hours, especially preferred between 2 hour and 12 hours, ideally between 3 and 6 hours. In the context of the present invention it has been found out that an oral administration with a retarded release comprising a duration of action between 4 and 5 hours is particularly effective, in particular for the therapy of infants. The retarded release can comprise an onset of activation, wherein the onset of activation describes the time interval between administration and the time the substance's effects come to prominence. The onset of activation can be between 1 second and 96 hours, preferably between 30 seconds and 12 hours, especially preferred between 1 minute and 2 hours, ideally between 5 minutes and 1 hour. In the context of the present invention it has been found out that an oral administration with a retarded release comprising an onset of activation between 20 and 40 minutes is particularly effective, in particular for the therapy of infants. It can be advantageous to achieve an bioavailability of at least 20%, in particular between 10% and 20% for oral administration, in particular to achieve an optimum effect. Bioavailability can describe the systemically available fraction of the substance.

Another aspect of the invention can be to provide a substance for use in the treatment of Trisomy 21, wherein the substance comprises at least one of the following excipients: amtodextrin, lactose, mannitol, starch (particularly corn starch or potato starch), crystalline cellulose, cellulose derivatives, acacia, gelatins, sodium carboxymethyl cellulose, talc, and/or magnesium stearate. This can at least partially provide a retarded release. The substance and/or the tablet can comprise between 0.01% to 99.9999%, preferably between 10% to 99.99%, especially preferred between 50% to 99%, ideally between 85% to 95% of the excipient, with respect to the total volume or mass. A large amount of the at least one excipient (compared to the substance), in particular between 95 and 99.9999%, can provide a cost efficient production. In an advantageous embodiment of the present invention, the substance comprises all of the aforementioned excipients. An excipient can be an inactive substance, in particular not having an effect on the subject, that is combined with an active substance to facilitate administration. The excipient can provide facilitated swallowing, digestion, additional nutrition, additional vitamins, additional electrolytes, improved long-term stabilization, improved compatibility, and/or improved skin compatibility.

Another aspect of the invention can be to provide a substance for use in the treatment of Trisomy 21, wherein the substance is administered to the substantially awake subject 3 drops per oz. a day, in particular during day time, advantageously during the wake hours during daytime, or during day and night time, advantageously during the wake hours both day and night time. An administration during the wake hours allows the subject to administer the substance itself. The substance can be administered every 96 hours. Preferably, the substance is administered every 48 hours, preferably every 24 hours. In other words, the dosing interval can comprise between 1 to 96 hours, preferably between 2 and 48 hours, especially preferred between 3 and 12 hours, ideally between 4 and 6 hours. The frequency of administration can depend on the variety and/or severity of Trisomy 21. An administration every 2 to 3 hours is likewise possible, and particularly beneficial for infants.

Another aspect of the invention can be to provide a substance for use in the treatment of Trisomy 21, wherein the substance is administered to the subject in substantially regular intervals, wherein the administration of the substance is performed in 1-96 intervals a day, advantageously 1-24 intervals a day, particularly advantageous 3-5 intervals a day, wherein in particular the substance is administered to the subject in 4 or 24 intervals a day. The more often the substance is administered to the subject, the more constant the plasma concentration and/or effect can be, which particularly can provide reduced symptoms and/or evolution of Trisomy 21.

Another aspect of the invention can be to provide a substance for use in the treatment of Trisomy 21, wherein the memory and/or cognition in a subject is improved, and wherein this particularly comprises administering to the subject a dose, in particular a therapeutically effective amount, of the substance in order to improve memory or cognition, or to treat a memory or cognitive disorder.

Another aspect of the invention can be to provide a substance for use in the treatment of Trisomy 21, wherein the substance is admitted prophylactically and/or therapeutically. Therapeutically can comprise the administration of the substance according to the aforementioned features. Prophylactically can comprise the administration of the substance according to the aforementioned features, in particular if the subject is not yet diagnosed with Trisomy 21. This can particularly comprise the administration to a fetus, advantageously by administration of the substance to the mother.

According to a second aspect of the invention, a tablet comprising a substance, in particular according to the first aspect of the invention, as described above and/or according to one of the claims 1 to 11, is provided, wherein the tablet at least partly suspenses within the mouth of a subject, and can subsequently be swallowed. In other words, a liberation of the substance at least partly from the tablet is performed. Advantageously, the tablet is more mechanically robust, can be stored longer, easier to administer, in particular easier to swallow, cheaper and/or easier to produce. Preferably, the tablet is administered to the subject, in particular the subject can comprise a human being, in particular a pregnant women, a fetus, a baby and/or an infant. The suspension can comprise the complete dissolution in the mouth of the subject. The tablet can suspend within the mouth of the subject in a suspension time between 1 and 600 seconds, preferably between 10 and 300 seconds, especially preferred between 20 and 180 seconds, ideally between 30 and 120 seconds. For infants a fast dissolution time can be preferred, due to the high chance of swallowing the tablet before suspension. A tablet can be seen as a form of a pharmaceutical dosage. The tablet can be a pill, capsule and variants thereof like a caplet (smooth, coated, oval-shaped medicinal tablet). The tablet can comprise a substance, in particular an active substance and/or excipients. A tablet can be generated in form of a powder, which is pressed or compacted into a solid dose. A tablet can be formulated to deliver a dose, in particular an accurate dosage to a specific site or tissue. The tablet can at least party be digested. Advantageously the tablet rapidly dissolves in saliva and/or is not dissolved until it is digested. The size of the tablet can be between 10µm and 2cm, preferably between 1mm and 1cm, especially preferred between 2mm and 8mm, ideally between 3mm and 5mm. This allows oral administration for adults and/or children, wherein a small size, in particular between 10µm to 3mm, is advantageous for children to prevent suffocation. The tablet can deliver a dose, in particular measured dosage, of the substance. Consequently, the tablet is suitable for applying the substance, analogues thereof, precursors thereof or its derivatives to the subject. Advantageously, the tablet is simple and convenient to use, preferably with rounded or chamfered edges to prevent injuries and/or prevent suffocation when swallowing the tablet.

Advantageously the tablet comprises at least one coating. A coating can be a thin layer around the tablet. Thereby the coating can comprise a sugar and/or a polymer and/or a polysaccharide base. Preferably plasticizers and pigments can be comprised. Thereby the coating can be stable, mechanically robust and/or strong enough to survive the handling of the tablet. Furthermore, a sticking together of tablets during storage, usage and/or administration is prevented. The coating can protect tablets from light, moisture and/or oxidation. A coating can make the tablet easier to swallow, allow for improved taste and/or mask odors. The tablet coating is further advantageously to enhance the stability of the tablet and protect against moisture or oxidation. An enteric coating can be used, which is resistant to stomach acid, and dissolves in the less acidic area of the intestines, in particular If the substance of the tablet is sensitive to acid, or is irritant to the stomach lining. The coating can be chosen in order to control the rate of dissolution of the substance in the gastrointestinal tract. A thickness of the coating between 1µm and 1cm, preferably between 5µm and 700µm, especially preferred between 20µm and 500µm, ideally between 50µm and 200µm. The coating can comprise between 0,1 to 25%, preferably between 0,5 to 10%, especially preferred between 1 to 5%, ideally between 2 to 3% of the initial weight of the tablet. A low percentage (e.g. 0,1 to 2,5%) can result in reduced costs since the coating can contain more expensive ingredients. A high percentage (e.g. 2,6 to 25%) can result in optimized mechanical robustness of the tablet and/or optimized properties for swallowing (e.g. a thicker coating film). The substance can be absorbed better at different points in the digestive system. If the highest percentage of absorption of the substance takes place in the stomach, a coating that dissolves quickly and easily in acid can be selected. If the rate of absorption is best in the large intestine or colon, then a coating that is acid resistant and dissolves slowly would be used to ensure it reached that point before dispersing.

The coating can comprise the substance, in particular partially. Thereby, the coating can comprise between 0.1% to 90%, preferably between 1% to 70%, especially preferred between 5% to 50%, ideally between 10% to 25% of the substance, with respect to the total volume or mass of the substance inside the tablet, in particular excluding the coating. This can lead to a raise of the plasma concentration since parts of the substance within the coating are already transferred to the blood, in particular while the tablet is in the mouth of the subject. Consequently, the increase in plasma concentration is less pronounced, which can particularly help to reduce the maximum plasma concentration, since preferably the amount of the substance inside the tablet can be reduced. This effect can be strong (more pronounced) for a large amount of the substance in the coating, and can be less pronounced for a small amount of the substance in the coating.

The tablet and/or coating can comprise crystalline cellulose, cellulose derivatives, acacia, gelatins, sodium carboxymethyl cellulose, talc, and/or magnesium stearate. The tablet and/or coating can comprise diluents, binders (or granulating agents), and/or glidants (or lubricants) to ensure efficient use as a tablet, improve the mechanical stability of the tablet and/or provide facilitated swallowing. The tablet can comprise disintegrants to at least partly promote tablet break-up in the digestive tract. Moreover also sweeteners or flavors can be used to enhance the taste and have a pleasant perception while intaking the tablet. Furthermore, pigments can be used in order to make the tablet visually attractive and/or distinguishable from other tablets, in particular with different dosage. The coating can provide polymers, in particular to provide a mechanical backbone, make the tablet smoother and/or easier to swallow, to control the release rate of the substance and/or to make it more resistant to the environment. The polymer can comprise cellulosics (such as hydroxypropyl methyl cellulose (HPMC), hydroxypropyl cellulose (HPC), and ethyl cellulose (EC)), vinyls (such as polyvinyl alcohol, acrylics (such as methacrylic acid / ethylacrylate copolymers, in particular for enteric or delayed release coating), and/or natural derivatives (such as shellac or alginates). The tablet can comprise lubricants comprising minerals (like talc or silica), and/or fats (e.g. vegetable stearin, magnesium stearate or stearic acid), in particular for improved swallowing and/or dissolving inside the mouth of the subject. The coating can comprise between 0.1% to 90%, preferably between 1% to 70%, especially preferred between 5% to 50%, ideally between 10% to 25% of the plasticizing agent, with respect to the total volume or mass of the coating. The coating can comprise a plasticizing agent, in particular to reduce the film-forming temperature of the polymer and/or make it possible to apply at lower temperatures, and/or to improve the elasticity of the coating. The plasticizing agent can comprise polyhydric alcohols (such as propylene glycol or polyethylene glycol (PEG) or glycerol), acetate esters (such as triacetin (glycerol triacetate) or triethyl citrate(TEC)), glycerides (such as acetylated monoglycerides) and/or oils (such as mineral oil or vegetable oils). The coating can comprise between 0.1% to 90%, preferably between 1% to 70%, especially preferred between 5% to 50%, ideally between 10% to 25% of the plasticizing agent, with respect to the total volume or mass of the coating.

The tablet and/or coating can comprise the substance and/or appropriate additives to make tablets, in particular for oral administration, powders, granules or capsules, for example, with conventional additives, such as lactose (for additional energy), mannitol, corn starch or potato starch, with binders (for improved mechanical robustness), such as crystalline cellulose, cellulose derivatives, acacia, corn starch or gelatins, with disintegrators, such as corn starch, potato starch or sodium carboxymethylcellulose, with lubricants (e.g. for facilitated swallowing), such as talc or magnesium stearate, hydrogenated vegetable oil, sodium stearyl fumarate, and/or with diluents (e.g. for improved dissolution properties and/or better tolerance), buffering agents, moistening agents, preservatives and flavoring agents (in particular to improve the acceptance for subjects, especially infants). The tablet can also comprise important nutrition, in particular vitamins, trace elements, salts, fiber, proteins, carbohydrates, and/or fats, especially unsaturated fatty acids, advantageously to support nutrition of the subject. At the same time, this can increase the thickness of the coating and thus result in a more robust tablet.

Alternatively or in addition, another aspect of the invention can be to provide a tablet, wherein the tablet comprises the substance in a single dosage and/or the tablet is wrapped in a blister package. In other words, the tablet can provide a distinct dosage of the substance, preferably in a convenient portable package. The tablet can be designed to protect unstable medications or disguise unpalatable ingredients. The tablet can be configured with colored coating, embossed markings, and/or a printing in order to aid tablet discrimination and recognition both for the subject, medical personnel and machines. Therefor a tablet can preferably be wrapped in a blister package, such that the content can preferably be seen and/or visually detected from outside without opening the package. This can optimize the technical production, delivery, storage, and/or reduce the risk of wrong application.

Alternatively or in addition, another aspect of the invention can be to provide a tablet, wherein the tablet is chewable. The tablet can be chewable as a tablet or as a gum. Chewable can mean that the tablet is based on an ion-exchange resin that releases the substance slowly when the subject chews, and particularly the substance present in the mouth is delivered directly to the systemic circulation and/or blood by buccal absorption and/or is swallowed and delivered to the intestinal tract, in particular peace by peace. Chewable can also comprise that the tablet and/or substance has a material hardness which still allows to chew on it without damaging the teeth.

According to a third aspect of the invention, a dispensing device for use with a substance, in particular as described above, according to the first aspect of the invention and/or according to one of the claims 1 to 11, or a tablet, in particular as described above, according to the second aspect of the invention and/or according to claim 12, is provided. Advantageously, the dispensing device provides for early, more robust, more effective, more reliable, and/or cheaper use with a substance in the treatment of Trisomy 21 and/or a tablet comprising the substance. The dispensing device comprises a housing with an air inlet means and a nozzle through which a dose of a substance entrained in an fluid flow is dispensed, and having means for actuating said dispensing device to dispense a measured dose of a substance. The fluid flow can enter the dispensing device, in particular via the air inlet, and can preferably comprise at least one gas and/or at least one liquid. The air inlet means can comprise simple holes and/or pipes, in particular to suck a gas and/or liquid from outside, advantageously air or oxygen, advantageously after passing a filter for cleaning. The air inlet means can provide gas, such that the subject does not have to work against a negative pressure during oral administration and/or inhalation. The nozzle can be a wind turbine or propeller in the simplest case. The nozzle can be used to provide the substance to a fluid flow, wherein the fluid flow is provided to the subject, in particular by oral administration and/or inhalation. Advantageously, the dispensing device can help the subject to administer a dose, in particular a correct dosage, in particular at certain points of time. This can lead to an optimized therapy, since the substance is sufficiently available in the subject at each point in time. The housing can be a simple cuboid, in particular comprising a grip which is molded to a human hand. The dispensing device can comprise pipes provided for fluid flow and/or a shelter provided to store a gas and/or fluid, in particular air, oxygen and/or water. The fluid flow can simply be a stream of gas and/or liquid essentially flowing through the housing, in particular due to pressure applied by a subject and/or a machine like a compressor. In other words, the dispensing device can be connected to the mouth of a subject, and the substance is combined and/or mixed with the fluid flow, wherein the fluid flow is transported to the mouth of the subject for inhalation and/or oral administration. The means for actuating can be used to activate the dispensing device. In particular, an administration of the substance and/or fluid flow is not possible, as long as the means for actuating is not used. In the simplest case, the means for actuating can be a reversible switch, which particularly has to be manually activated against a preload, which can advantageously be provided by a spring.

The dispensing device can also comprise an automated dispensing cabinet comprising a computerized medication, wherein a substance can be stored and/or dispensed near the subject, wherein the dispensing of the substance is controlled and tracked. The housing can therefore be a tower and/or rack unit, in particular to fit in a 19 or 23 inch rack frame.

Another aspect of the invention can be to provide a dispensing device, wherein the substance is dispensed in a predefined dosage, in particular comprising a single dosage or a double dosage. In other words, only a predefined dosage is administered to the subject, in particular when the means for actuating is used. The predefined dosage can be a single and/or double dosage. The double dosage can comprise a dose which is two times as high as the single dosage. The subject can therefore decide if a single or double dosage should be administered, for example by using the means for actuating once for a single dosage or twice for a double dosage. The dispensing device can comprise a control unit connected to the means for actuating, in particular a computer and/or integrated circuit which can be programmed, in particular to save a dosing interval which can be predefined and/or a predefined dosage. The control unit can be programmed to allow the administration of the substance only in dosing intervals, in order to provide a controlled administration and prevent high dose or overdose. Alternatively or in addition, the control unit can be programmed to administer a predefined dosage, e.g. a predefined volume or mass of the substance. The control unit can also provide for a controlled fluid flow, in particular by controlling the speed of. Consequently, the subject does not need to control the dosage and/or fluid flow.

Another aspect of the invention can be to provide a dispensing device, wherein the dispensing device is automated, in particular, that a dosing interval in which a substance is dispensed can be manipulated, wherein the dosing interval is advantageously equidistant in time. The control unit can be used to manipulate the dosing interval, in particular to provide equidistant dosing intervals. In other words, the points in time at which the substance can be administered using the dispensing device is predefined, in particular by the control unit. The control unit can be programmed to realize different user profiles. This can comprise different subjects to use the dispensing device, and advantageously providing each subject with the correct predefined dosage.

Further features and details of the invention result from the claims and/or the description. Features and details which have been described in connection with the substance according to the invention (in particular the first aspect of the invention) naturally also apply in connection (or can be comprised) with the tablet according to the invention (in particular the second aspect of the invention) and/or the dispensing device according to the invention (in particular the third aspect of the invention) and vice versa in each case, so that with regard to the disclosure concerning the individual aspects of the invention reference is or can always be made mutually. Features discussed within the description of the examples and/or the figures can be freely combined with each other. It is shown:

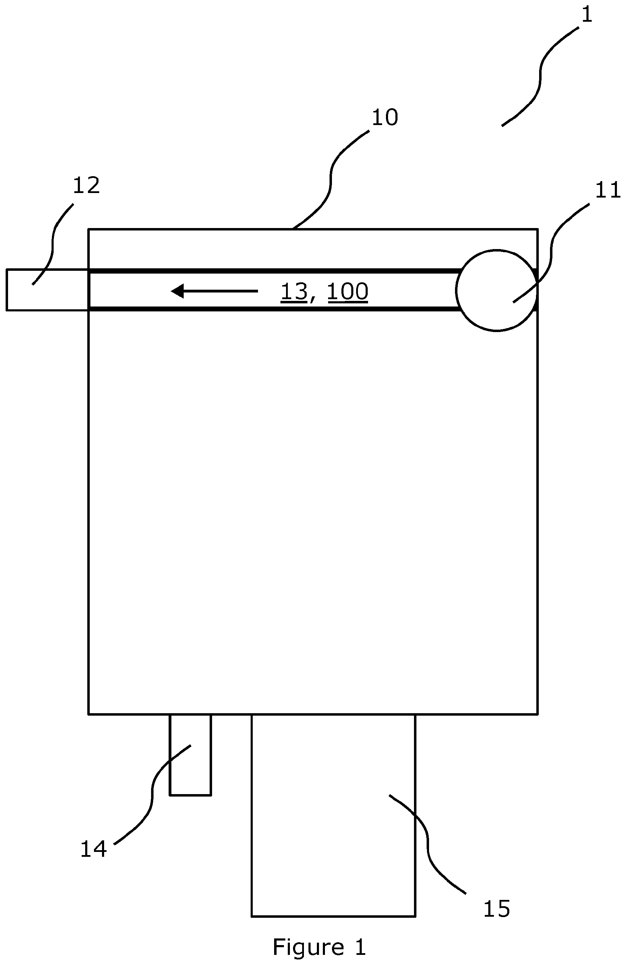

Fig. 1 an embodiment of a dispensing device according to the invention.

### Description of the Figure

Figure 1 shows a dispensing device (1) comprising a housing (10) with an air inlet means (11) and a nozzle (12) through which a dose of a substance (100) entrained in an fluid flow (13) is dispensed, and having means for actuating (14) said dispensing device (1) to dispense a measured dose of a substance (100). The fluid flow (13) can enter the dispensing device (1), in particular via the air inlet (11), and can preferably comprise at least one gas and/or at least one liquid. The air inlet means (11) can comprise simple holes and/or pipes, in particular to suck a gas and/or liquid from outside, advantageously air or oxygen, advantageously after passing a filter for cleaning. The air inlet means (11) can provide gas, such that the subject does not have to work against a negative pressure during oral administration and/or inhalation. The nozzle (12) can be a wind turbine or propeller in the simplest case. The nozzle (12) can be used to provide the substance to a fluid flow (13), wherein the fluid flow (13) is provided to the subject, in particular by oral administration and/or inhalation. Advantageously, the dispensing device (1) can help the subject to administer a dose, in particular a correct dosage, in particular at certain points of time. This can lead to an optimized therapy, since the substance is sufficiently available in the subject at each point in time. The housing (10) can be a simple cuboid, in particular comprising a grip (15) which is molded to a human hand. The dispensing device can comprise pipes provided for fluid flow and/or a shelter provided to store a gas and/or fluid, in particular air, oxygen and/or water. The fluid flow (13) can simply be a stream of gas and/or liquid essentially flowing through the housing (10), in particular due to pressure applied by a subject and/or a machine like a compressor. In other words, the dispensing device can be connected to the mouth of a subject, and the substance is combined and/or mixed with the fluid flow (13), wherein the fluid flow (13) is transported to the mouth of the subject for inhalation and/or oral administration. The means for actuating (14) can be used to activate the dispensing device (1). In particular, an administration of the substance (100) and/or fluid flow (13) is not possible, as long as the means for actuating (14) is not used. In the simplest case, the means for actuating (14) can be a reversible switch, which particularly has to be manually activated against a preload, which can advantageously be provided by a spring.

### Reference list

- 1: dispensing device
- 10: housing
- 11: air inlet means
- 12: nozzle
- 13: fluid flow
- 14: means for actuating
- 15: grip
- 100: substance

## Claims

1. Substance, analogues thereof, precursors thereof, or its derivates, for use in the treatment of Trisomy 21,
**characterized in that,**
the substance comprises C₁₇H₂₀N₂O₃,
a compound A, or a pharmaceutically acceptable substitute in particular a salt thereof 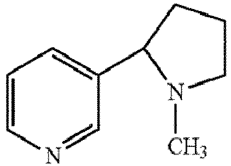
and/or
a compound B, or a pharmaceutically acceptable substitute in particular a salt thereof

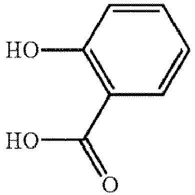

2. Substance for use in the treatment of Trisomy 21 according to claim 1, **characterized in that,**
the substance is designed for postnatal administration, in particular of newborn babies and infants, and/or for prenatal treatment of a pregnant human and/or the fetus.

3. Substance for use in the treatment of Trisomy 21 according to one of the preceding claims,
**characterized in that,**
the substance is essentially administered in solid, liquid or gaseous form or in an essentially hybrid form thereof.

4. Substance for use in the treatment of Trisomy 21 according to one of the preceding claims,
**characterized in that,**
the compounds A and B are substantially at a ratio of between 10:1 to 1:10, by weight, particularly at a ratio of about 1.5:1.

5. Substance for use in the treatment of Trisomy 21 according to one of the preceding claims,
**characterized in that,**
the substance is adapted for oral administration, for inhalation, nasal administration, intravenous administration, and/or adhesive administration,
wherein oral administration in particular comprises that the substance is essentially shaped as a tablet, as a suppository or as an essentially shaped granulate, wherein advantageously the oral administration is essentially buccal, sublabial or sublingual, wherein in particular adhesive administration comprises a substantially gel-like substrate, advantageously such as an ointment, lotion, cream, gel, or tincture.

6. Substance for use in the treatment of Trisomy 21 according to one of the preceding claims,
**characterized in that,**
the substance is designed for the affection of nAch receptors, in particular that the substance couples to said nAch receptors and/or
the substance is designed to change, in particular to increase or accelerate, at least one of the following parameters, the rate of cell growth or cell division in order to change the proportion of abnormal cells compared to normal cells, in particular that the amount of normal cells is higher than the amount of abnormal cells.

7. Substance for use in the treatment of Trisomy 21 according to one of the preceding claims,
**characterized in that,**
the substance is designed to change, in particular to reduce or heal, at least one of the following parameters, tiredness, crying, sickness, macroglossia, hypotonia, cognitive dysfunction, cognitive impairment, congenital heart diseases, mental impairment, stunted growth, increased skin on back of the neck, roof of mouth, flat head, flexible ligaments, protruding tone, abnormal outer ears, flattened nose, abnormal teeth, slanted eyes, short neck, shortened hands, obstructive sleep apnea, single transverse palmar crease, Brushfield spots in the iris, strabismus, undescended testicles, leukemia, Hypothyroidism, Constipation, Gingivitis, Infertility, Alzheimer's disease, accelerated aging, cyanotic color or bruising or crusting or hypotonic tendencies of body parts, in particular of the lips, the fingers, and the nails.

8. Substance for use in the treatment of Trisomy 21 according to one of the preceding claims,
**characterized in that,**
the substance is adapted for retarded release.

9. Substance for use in the treatment of Trisomy 21 according to one of the preceding claims,
**characterized in that,**
the substance comprises at least one of the following excipients: amtodextrin, lactose, mannitol, starch, particularly corn starch or potato starch, crystalline cellulose, cellulose derivatives, acacia, gelatins, sodium carboxymethyl cellulose, talc, magnesium stearate.

10. Substance for use in the treatment of Trisomy 21 according to one of the preceding claims,
**characterized in that,**
the substance is administered to the substantially awake subject 3 drops per oz. a day, in particular during day time, advantageously during the wake hours during daytime, or during day and night time, advantageously during the wake hours both day and night time.

11. Substance for use in the treatment of Trisomy 21 according to one of the preceding claims,
**characterized in that,**
the substance is administered to the subject in substantially regular intervals, wherein the administration of the substance is performed in 1-96 intervals a day, advantageously 1-24 intervals a day, particularly advantageous 3-5 intervals a day, wherein in particular the substance is administered to the subject in 4 or 24 intervals a day.

12. Tablet comprising a substance according to one of the claims 1 to 11,
**characterized in that,**
the tablet essentially suspenses within the mouth of a subject, and/or
the tablet comprises the substance in a single dosage and/or
the tablet is wrapped in a blister package and/or
the tablet is chewable.

13. Dispensing device for use with a substance according to one of the claims 1 to 11, or a tablet according to claim 12,
**characterized in that,**
the dispensing device comprises a housing with an air inlet means and a nozzle through which a dose of the substance entrained in an fluid flow is dispensed, and having means for actuating said dispensing device to dispense a measured dose of the substance.

14. Dispensing device according to claim 13,
**characterized in that,**
the substance is dispensed in a predefined dosage, in particular comprising a single dosage or a double dosage.

15. Dispensing device according to claim 13 or 14,
**characterized in that,**
the dispensing device is automated, in particular, that a dosing interval in which a substance is dispensed can be manipulated, wherein the dosing interval is advantageously equidistant in time.
